# FASCICULE DE BREVET EUROPEEN

(11) **EP 3 223 686 B1**
(45) Date de publication et mention de la délivrance du brevet: **26.05.2021**
(21) Numéro de dépôt: 15821164.9
(22) Date de dépôt: 24.11.2015
(51) Int. Cl.: A61B 5/00

(54) **DISPOSITIF DE STIMULATION VIBROTACTILE**
VORRICHTUNG ZUR VIBROTAKTILEN STIMULATION
VIBROTACTILE STIMULATION DEVICE

(30) Priorité: 24.11.2014 FR 1461377
(43) Date de publication de la demande: 04.10.2017
(73) Titulaire: INSERM - Institut National de la Santé et de la Recherche Médicale, 75013 Paris (FR); Université de Rennes I, 35000 Rennes (FR)
(72) Inventeur: HERNANDEZ, Alfredo, F-35510 Cesson-Sevigne (FR)
(74) Mandataire: Le Forestier, Eric
(86) Numéro de dépôt international: PCT/IB2015/059083
(87) Numéro de publication internationale: WO 2016/083998

(56) Documents cités:
- US-A1- 2002 029 924
- US-A1- 2007 232 962
- US-A1- 2013 102 937
- US-A1- 2014 046 166

## Description

### Domaine de l'invention

La présente invention concerne d'une façon générale les dispositifs de stimulation vibrotactile ou kinesthésique.

### Etat de la technique

La stimulation vibrotactile ou kinesthésique est connue dans l'état de l'art et couramment utilisée depuis plus de 20 ans dans différents domaines d'application, notamment, pour l'assistance sensorielle.

Ainsi le document US 5 035 242 A vise un dispositif de stimulation à l'usage des malentendants, le document WO 2009082682 A1 propose un dispositif pour les personnes avec déficience visuelle.

La stimulation vibrotactile est encore utilisée pour le traitement des apnées du sommeil, notamment chez le nouveau-né prématuré (voir en particulier US 5 555 891 A ou WO2007141345 A1).

Plusieurs principes de transduction sont utilisés dans les effecteurs vibro-tactiles ou kinesthésiques. Dans leur grande majorité, ils délivrent une stimulation mécanique à partir de l'application d'un signal électrique de caractéristiques prédéfinies.

Indépendamment du type de transduction électromécanique utilisée, l'énergie mécanique produite par l'effecteur est transmisse dans le milieu cible (peau, os, etc.) d'une façon qui est largement dépendante de la qualité du couplage entre l'effecteur et ledit milieu cible.

Ainsi un dispositif de stimulation paramétré pour appliquer une stimulation d'énergie donnée risque, du fait d'une mauvaise qualité de couplage, de délivrer au milieu cible une énergie insuffisante pour atteindre l'effet désiré, ce qui dans certains cas peut conduire à une prolongation d'une phase d'apnée de façon dramatique pour le patient. Plus généralement, compte-tenu des incertitudes sur la qualité du couplage, le système de stimulation ne peut pas garantir qu'il a effectivement délivré la thérapie souhaitée.

Le document US2013102937A1 décrit un dispositif pour le traitement de l'hypertension, où un traitement vibratoire est appliqué dans certaines régions. Un capteur d'impédance est prévu pour indiquer si le dispositif est placé correctement sur le corps de l'utilisateur.

Pour mémoire, le document US2007232962A1 décrit un dispositif de traitement thérapeutique à base de vibration se propageant en surface dans la peau.

Enfin pour mémoire également le document US2002029924A1 décrit un dispositif de mesure des propriétés élastiques en surface d'une structure.

### Résumé de l'invention

La présente invention vise à proposer un dispositif de stimulation vibrotactile, notamment mais non exclusivement pour le traitement de l'apnée du sommeil, qui soit capable d'estimer de façon réaliste la qualité du couplage mécanique avec le milieu cible, et si nécessaire d'ajuster l'énergie vibrotactile fournie par l'effecteur pour atteindre l'effet requis sur le patient.

On propose ainsi selon l'invention un dispositif de stimulation vibrotactile tel que défini dans la revendication 1.

On pourra compléter les caractéristiques de la revendication 1 avec les caractéristiques préférées mais optionnelles des revendications dépendantes.

### Brève description des dessins

D'autres aspects, buts et avantages de la présente invention apparaîtront mieux à la lecture de la description détaillée suivante d'une forme de réalisation préférée de celle-ci, donnée à titre d'exemple non limitatif et faire en référence aux dessins annexés, sur lesquels :
La Figure 1 est une vue de face schématique d'un dispositif de stimulation vibrotactile, destiné à se placer en arrière de l'oreille d'un patient, et des différents éléments constitutifs de ce dispositif, et
La Figure 2 est une vue en coupe transversale schématique et partielle du dispositif de la Figure 1, et
La Figure 3 est une vue en coupe transversale d'un exemple d'implémentation pratique du dispositif des Figures 1 et 2.

### Description détaillée d'une forme de réalisation préférée

En référence aux dessins, un dispositif de stimulation vibrotactile ou kinesthésique 1 selon l'invention comprend de façon fondamentale un effecteur ou excitateur électromécanique vibratoire 10 et un capteur de mouvements 20.

De façon préférée, l'effecteur 10 comprend un organe piézo-électrique ou un actuateur linéaire résonant, tandis que le capteur 20 comprend un accéléromètre, de préférence un accéléromètre trois axes. Ce dispositif comprend également des moyens de traitement numérique embarqués 30, typiquement sous la forme d'une carte électronique dotée d'un microcontrôleur, destinés à traiter les signaux de stimulation appliqués à l'effecteur et les signaux recueillis par le capteur 20 de manière à estimer la qualité du couplage entre l'effecteur et le milieu cible MC (en l'espèce la région sous-cutanée du patient en arrière d'une oreille) et ainsi l'efficacité de l'énergie mécanique délivrée par l'effecteur au milieu cible.

Le dispositif peut être doté d'autres fonctionnalités. Il peut par exemple intégrer un capteur de température 40 délivrant des signaux de température soit analogiques et convertis en signaux numériques au niveau du dispositif de traitement, doit directement numériques, un dispositif 50 de signalisation lumineuse (DEL) et/ou sonore (vibreur) exprimant l'état du dispositif, un interrupteur de marche/arrêt, etc.

Il est alimenté par une pile bouton 60 ayant une capacité appropriée, ou éventuellement une batterie rechargeable, de façon filaire (par exemple sur le port USB D'un ordinateur) ou sans fil (par transmission inductive de puissance, de façon connue en soi pour les petits appareils électroniques).

Selon une variante de réalisation, les moyens de traitement numérique, ou une partie de ces moyens de traitement, peuvent être déportés dans un boîtier distinct du dispositif et soit porté par le patient, soit disposé à son voisinage, par exemple sur sa table de nuit, pendant le sommeil.

Des moyens de transmission sont alors prévus pour permettre la communication du dispositif avec le boîtier déporté, ces moyens pouvant être filaires ou sans fil pour un boîtier porté par le patient, et de préférence sans fil pour un boîtier fixe.

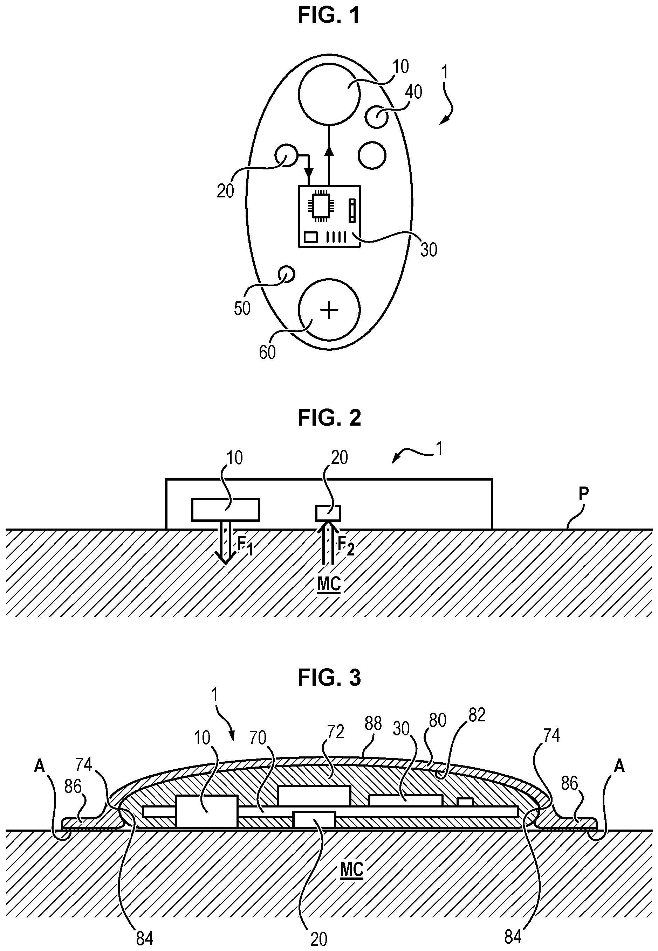

En référence plus particulièrement à la Figure 2, le dispositif est apte à émettre, en fonction de critères tels que la détection d'un phénomène d'apnée du sommeil par des moyens non décrits ici et pouvant être conventionnels (par exemple la détection d'un flux d'air respiratoire ou d'un mouvement respiratoire), des impulsions mécaniques d'excitation à l'aide de l'effecteur 10. Ces impulsions ont par exemple une fréquence de l'ordre de 100 à 400 Hz, et sont commandés par le circuit de commande 30 en réponse aux signaux de détection reçus via une interface filaire ou sans fil non illustrée.

La flèche F1 illustre l'énergie mécanique vibratoire transmise au milieu MC, qui comprend une composante principale perpendiculaire à l'interface physique entre le dispositif et le milieu, mais également des composantes, d'amplitude plus faible, dans les deux dimensions du plan de cette interface.

Une partie de cette énergie vibratoire est dispersée latéralement par le milieu MC, et la flèche F2 illustre une fraction de cette énergie située au droit du capteur 20. Ce dernier recueille un signal, fourni au circuit 30, représentatif de la façon dont le milieu MC a modifié l'énergie mécanique vibratoire injectée par l'effecteur.

On comprend que l'amplitude et le contenu fréquentiel du signal recueilli par le capteur 20 dépendent directement de la qualité du couplage entre l'effecteur 10 et le milieu MC. En effet, le milieu MC peut être vu comme une fonction de transfert linéaire ou non-linéaire entre le signal d'entrée (signal de stimulation) et le signal recueilli par le capteur 20. Le circuit 30 dispose donc en temps réel ou quasi-réel d'une information relative à la qualité de ce couplage. Il est donc capable notamment :
- de moduler les paramètres du signal appliquée à l'effecteur 10, et notamment d'amplifier les impulsions vibratoires dans le cas d'un mauvais couplage ; par exemple, on peut mettre en oeuvre une loi mathématique simple consistant à augmenter l'énergie appliquée par l'effecteur d'un facteur 1/X pour un taux de transmission X (inférieur à 1 et mesuré par rapport à la transmission par un milieu « idéal ») ;
- de détecter et signaler, par exemple à l'aide du dispositif de signalisation 50, que le dispositif est mal couplé au milieu, en détectant que l'énergie captée au niveau de la flèche F2, pour une énergie injectée dans le milieu selon la flèche F1, est inférieure à un certain seuil.

Il est à observer ainsi que la mesure de l'énergie propagée par le milieu MC à l'aide du détecteur accélérométrique 20 peut permettre de donner à l'utilisateur un indicateur de pose correcte du dispositif 1. Notamment on peut prévoir que le dispositif, après sa pose et sa mise en route (à l'aide d'un bouton marche-arrêt par exemple), génère un train d'impulsions d'énergie donnée, et ne valide la pose que lorsque l'énergie recueillie au niveau du capteur 20 est supérieure à un certain seuil, ou encore supérieure à un certain seuil minimum et inférieure à un certain seuil maximum.

On notera également que la détection du couplage entre le dispositif 1 et le milieu cible MC et/ou la détection d'une pose correcte peut être effectuée, ou rendue plus fine, en exploitant également les valeurs d'amplitude et du contenu fréquentiel des signaux vibratoires recueillis par le capteur 20 selon les deux axes (notés x et y) contenus dans le plan d'interface dispositif/milieu, en plus de la valeur d'amplitude et de contenu fréquentiel selon l'axe perpendiculaire z.

En particulier, on a observé que la transmission de l'énergie vibratoire sous forme d'ondes de surface variait d'une façon qui est bien représentative de la pression exercée par l'effecteur 10 sur le milieu cible, en l'occurrence de la surface de la peau.

Les dimensions du dispositif sont typiquement de 3 à 8 cm selon le grand axe et de 2 à 6 cm selon le petit axe, et la distance entre l'axe de l'effecteur 10 et l'axe du capteur 20 est préférentiellement comprise entre 0,5 et 3 cm pour éviter une trop grande dispersion de l'énergie transmise. Ces valeurs ne sont toutefois nullement limitatives.

La Figure 3 illustre une implémentation pratique du dispositif selon l'invention. Les différents éléments du dispositif sont montés sur une platine 70, l'ensemble étant enrobé dans un bloc ou coque 72 en résine qui dispose de bords périphériques 74 appropriés au couplage avec un moyen de fixation à la peau. Un tel moyen de fixation peut être une enveloppe jetable 80, réalisée en un matériau élastique et capable de recevoir le dispositif dans une cavité interne 82 en retenant celui-ci par les bords 74 du bloc de résine d'encapsulation, engagés dans une encoche périphérique 84 de ladite enveloppe.

Un adhésif biocompatible A peut être prévu sur le bord périphérique 86 de ladite enveloppe, destiné à être en contact avec la peau, tandis qu'une paroi de dessus 88 de l'enveloppe recouvre et étanchéifié entièrement le dispositif encapsulé dans son bloc de résine 72.

On comprend que le dispositif 1 encapsulé peut être aisément extrait de l'enveloppe 80 pour la remplacer par une enveloppe neuve. L'adhésif A peut être couvert, de façon classique en soi, d'une pellicule de protection pelable à retirer avant usage.

Le dispositif de stimulation selon l'invention peut être fixé sur tout site adapté (derrière l'oreille, sur le thorax latéral, la plante des pieds, etc., l'enveloppe 80 et ses caractéristiques étant alors adaptées à l'usage prévu.

La Figure 3 montre que la base du dispositif, où débouchent l'effecteur 10 et le capteur 20, est en contact avec la surface du milieu à stimuler, en l'occurrence la peau via le matériau de la coque 72, choisi pour assurer un couplage mécanique approprié avec le milieu MC.

Les modifications suivantes sont envisagées :
- l'effecteur 10 et le capteur 20 peuvent être prévus sur une même unité ou sur deux unités distinctes prévus à proximité l'une de l'autre sur le site à stimuler ;
- il est possible d'exploiter les signaux fournis par le capteur 20, notamment lorsqu'il s'agit d'un accéléromètre trois axes, pour déterminer la position du site d'implantation, et notamment l'orientation de la tête du sujet lorsque le dispositif est disposé derrière l'oreille ; ceci permet avantageusement d'adapter les stratégies de stimulation, notamment pour le traitement de l'apnée du sommeil, en fonction des caractéristiques des phénomènes d'apnée rencontrés et/ou de la position du patient ; un gyroscope permet en variante de réaliser la même fonctionnalité ;
- on peut adjoindre au dispositif toute fonctionnalité indépendante ou corrélée à la stimulation vibratoire, et notamment, outre la mesure de température, toute détection ou captage d'un paramètre biologique ;
- lors de la détection d'un couplage non satisfaisant entre l'effecteur et le milieu à stimuler, on peut prévoir d'ajuster non seulement l'énergie des impulsions d'énergie mécanique vibratoire, mais également d'autres paramètres tels que leur forme d'onde ou leur fréquence.

## Revendications

1. Dispositif (1) de stimulation vibrotactile, notamment pour effectuer une stimulation corporelle dans la lutte contre les apnées du sommeil, destiné à être appliqué contre un milieu corporel (MC) à stimuler et comprenant un effecteur vibrant (10) apte à appliquer audit milieu des impulsions d'énergie mécanique vibratoire, et des moyens (30) de commande de l'effecteur selon des règles de stimulation, et des moyens détecteurs (20) aptes à être couplés mécaniquement au milieu corporel pour recevoir une part de énergie vibratoire transmise vers ces moyens par ledit milieu lors de l'application des impulsions d'énergie vibratoire, et déterminer la qualité du couplage entre l'effecteur et le milieu à stimuler, lesdits moyens détecteurs étant reliés aux moyens de commande, et
le dispositif étant **caractérisé en ce qu'**il comprend en outre des moyens (30) pour ajuster les paramètres des impulsions appliquées par l'effecteur en fonction de la qualité du couplage en amplifiant les impulsions vibratoires dans le cas d'un mauvais couplage.

2. Dispositif selon l'une des revendication 1, **caractérisé en ce que** l'effecteur (10) et les moyens détecteurs (20) sont reçus dans une unité commune.

3. Dispositif selon l'une des revendications 1 et 2, **caractérisé en ce que** les moyens détecteurs (20) comprennent un accéléromètre.

4. Dispositif selon l'une des revendications 1 à 3, **caractérisé en ce que** les moyens détecteurs (20) sont aptes à effectuer une détection cinématique selon un axe correspondant à un axe de vibration privilégié de l'effecteur.

5. Dispositif selon l'une des revendications 1 à 4, **caractérisé en ce que** l'effecteur (10) est apte à transmettre une énergie vibratoire selon un axe principal perpendiculaire à une interface entre l'effecteur et le milieu à stimuler.

6. Dispositif selon les revendications 3 à 5 prises en combinaison, **caractérisé en ce que** l'accéléromètre (20) est un accéléromètre à au moins deux axes dont l'un est généralement parallèle audit axe principal.

7. Dispositif selon l'une des revendications 1 à 6, **caractérisé en ce qu'**il comprend des moyens (30) aptes, en réponse aux signaux fournis par les moyens détecteurs, à délivrer une information de pose correcte et/ou incorrecte du dispositif sur le milieu à stimuler.

8. Dispositif selon l'une des revendications 1 à 7, **caractérisé en ce qu'**il comprend une platine (70) portant ses différents organes, et une enveloppe souple jetable (80) apte à recevoir la platine de façon amovible.

9. Dispositif selon la revendication 8, **caractérisé en ce que** l'ensemble des organes portés par la platine (70) sont encapsulés.

10. Dispositif selon la revendication 8 ou 9, **caractérisé en ce que** l'enveloppe souple (80) comporte des aménagements (84) aptes à recevoir et retenir élastiquement au moins une partie de bord du dispositif.

11. Dispositif selon l'une des revendications 8 à 10, **caractérisé en ce que** ladite enveloppe (80) présente un bord périphérique (86) pourvu d'un adhésif (A).

## Patentansprüche

1. Vorrichtung (1) zur vibrotaktilen Stimulation, insbesondere zum Durchführen einer Körperstimulation bei der Bekämpfung von Schlafapnoe, die auf eine zu stimulierende Körpergegend (MC) aufgelegt werden soll und einen Vibrationseffektor (10), der geeignet ist, auf die Gegend Impulse mechanischer Schwingungsenergie aufzubringen, und Mittel (30) zum Steuern des Effektors gemäß Stimulationsregeln und Detektormittel (20) umfasst, die geeignet sind, mechanisch mit der Körpergegend gekoppelt zu werden, um einen Teil der Schwingungsenergie aufzunehmen, die beim Aufbringen von Schwingungsenergieimpulsen von diese Gegend auf diese Mittel übertragen wird, und die Qualität der Kopplung zwischen dem Effektor und der zu stimulierenden Gegend zu bestimmen, wobei die Detektormittel mit den Steuermitteln verbunden sind und die Vorrichtung **dadurch gekennzeichnet ist, dass** sie ferner Mittel (30) zum Einstellen der Parameter der vom Effektor aufgebrachten Impulse in Abhängigkeit von der Qualität der Kopplung durch Verstärken der Vibrationsimpulse im Falle einer mangelhaften Kopplung umfasst.

2. Vorrichtung nach einem der Ansprüche 1, **dadurch gekennzeichnet, dass** der Effektor (10) und die Detektormittel (20) in einer gemeinsamen Einheit aufgenommen sind.

3. Vorrichtung nach einem der Ansprüche 1 und 2, **dadurch gekennzeichnet, dass** die Detektormittel (20) einen Beschleunigungsmesser umfassen.

4. Vorrichtung nach einem der Ansprüche 1 bis 3, **dadurch gekennzeichnet, dass** die Detektormittel (20) geeignet sind, eine kinematische Erfassung entlang einer Achse durchzuführen, die einer bevorzugten Schwingungsachse des Effektors entspricht.

5. Vorrichtung nach einem der Ansprüche 1 bis 4, **dadurch gekennzeichnet, dass** der Effektor (10) geeignet ist, Schwingungsenergie entlang einer zu einer Grenzfläche zwischen dem Effektor und der zu stimulierenden Gegend senkrechten Hauptachse zu übertragen.

6. Vorrichtung nach den kombinierten Ansprüchen 3 bis 5, **dadurch gekennzeichnet, dass** der Beschleunigungsmesser (20) ein Beschleunigungsmesser mit mindestens zwei Achsen ist, von denen eine im Allgemeinen parallel zu der Hauptachse ist.

7. Vorrichtung nach einem der Ansprüche 1 bis 6, **dadurch gekennzeichnet, dass** sie Mittel (30) umfasst, die geeignet sind, als Reaktion auf die von den Detektormitteln gelieferten Signale eine Information über die korrekte und/oder inkorrekte Lage der Vorrichtung auf der zu stimulierenden Gegend zu liefern.

8. Vorrichtung nach einem der Ansprüche 1 bis 7, **dadurch gekennzeichnet, dass** sie eine Platine (70) umfasst, die ihre verschiedenen Elemente trägt, und eine flexible Einweghülle (80), die geeignet ist, die Platine entnehmbar aufzunehmen.

9. Vorrichtung nach Anspruch 8, **dadurch gekennzeichnet, dass** alle von der Platine (70) getragenen Elemente verkapselt sind.

10. Vorrichtung nach Anspruch 8 oder 9, **dadurch gekennzeichnet, dass** die flexible Hülle (80) Einrichtungen (84) umfasst, die geeignet sind, mindestens einen Randabschnitt der Vorrichtung aufzunehmen und elastisch festzuhalten.

11. Vorrichtung nach einem der Ansprüche 8 bis 10, **dadurch gekennzeichnet, dass** die Hülle (80) eine Umfangskante (86) aufweist, die mit einem Klebemittel (A) versehen ist.

## Claims

1. A vibrotactile stimulation device (1), especially for performing a bodily stimulation in the fight against sleep apnea, adapted for be applied against a body medium (MC) to be stimulated and comprising a vibrating effector (10) capable of applying to said medium pulses of mechanical vibrational energy, and means (30) for controlling the effector according to stimulation rules, and detector means (20) adapted for being mechanically coupled to the body medium for receiving a portion of the vibrational energy transmitted toward these means by said medium during the application of the vibrational energy pulses, and for determining the coupling quality between the effector and the medium to be stimulated, said detector means being connected to the control means, and the device being **characterized in that** it further comprises means (30) for adjusting the parameters of the pulses applied by the effector as a function of the coupling quality, amplifying the vibrational pulses in the case of a bad coupling.

2. The device as claimed in claim 1, **characterized in that** the effector (10) and the detector means (20) are received in a common unit.

3. The device as claimed in one of claims 1 and 2, **characterized in that** the detector means (20) comprise an accelerometer.

4. The device as claimed in one of claims 1 to 3, **characterized in that** the detector means (20) are capable of effecting a kinematic detection along an axis corresponding to a preferred vibrational axis of the effector.

5. The device as claimed in one of claims 1 to 4, **characterized in that** the effector (10) is capable of transmitting a vibrational energy along a main axis perpendicular to an interface between the effector and the medium to be stimulated.

6. The device as claimed in one of claims 3 to 5 taken in combination, **characterized in that** the accelerometer (20) is an accelerometer with at least two axes, one of which is generally parallel to said main axis.

7. The device as claimed in one of claims 1 to , **characterized in that** it comprises means (30) capable, in response to the signals provided by the detector means, of providing information as to correct and/or incorrect placement of the device on the medium to be stimulated.

8. The device as claimed in one of claims 1 to 7, **characterized in that** it comprises a board (70) carrying its various members, and a disposable flexible casing (80) capable of receiving the board in a removable manner.

9. The device as claimed in claim 8, **characterized in that** all the members carried by the board (70) are encapsulated.

10. The device as claimed in claim 8 or 9, **characterized in that** the flexible casing (80) comprises arrangements (84) capable of receiving and resiliently holding at least an edge portion of the device.

11. The device as claimed in one of claims 8 to 10, **characterized in that** said casing (80) has a peripheral edge (86) provided with an adhesive (A).
